# EUROPEAN PATENT SPECIFICATION

(11) **EP 1 485 334 B1**
(45) Date of publication and mention of the grant of the patent: **27.05.2009**
(21) Application number: 03744344.7
(22) Date of filing: 06.03.2003
(51) Int. Cl.: C07C 2/58

(54) **ALKYLATION OF SATURATED HYDROCARBONS USING INTERSTAGE DISTILLATION**
ALKYLIERUNG GESÄTTIGTER KOHLENWASSERSTOFFE MIT EINER DESTILLATION ZWISCHEN DEN VERFAHRENSSTUFEN
ALKYLATION D'HYDROCARBURES SATURES PAR DISTILLATION INTERETAGE

(30) Priority: 18.03.2002 US 365346 P; 26.06.2002 EP 02077595
(43) Date of publication of application: 15.12.2004
(73) Proprietor: Albemarle Netherlands B.V., 3818 LH Amersfoort (NL); ABB LUMMUS GLOBAL INC., Bloomfield, New Jersey 07003 (US)
(72) Inventor: NAT, Pieter, Jan, NL-1035 PP Amsterdam (NL); VAN BROEKHOVEN, Emanuel, Hermanus, NL-1141 DN Monnickendam (NL); SONNEMANS, Johannes, Wilhelmus, Maria, NL-3761 DD Soest (NL); D'AMICO, Vincent, James, Glen Ridge, NJ 07028 (US); MUKHERJEE, Mitrajit, East Hanover, NJ 07936 (US)
(74) Representative: Rasser, Jacobus Cornelis
(86) International application number: PCT/EP2003/002367
(87) International publication number: WO 2003/078360

(56) References cited:
- US-A- 2 406 709

## Description

This invention relates to a continuous process for the alkylation of saturated hydrocarbons, in general branched saturated hydrocarbons, with an olefin to give highly branched saturated hydrocarbons with a higher molecular weight.
Within the framework of the present invention, the term alkylation refers to the reaction of an alkylatable compound, i.e. a saturated hydrocarbon, with an alkylation agent, such as an olefin.

In particular, this reaction is of interest because it makes it possible to obtain, through the alkylation of isobutane with an olefin containing 2-6 carbon atoms, an alkylate which has a high octane number and which boils in the gasoline range. Unlike gasoline obtained by cracking heavier petroleum fractions such as vacuum gas oil and atmospheric residue, gasoline obtained by alkylation is essentially free of contaminants such as sulphur and nitrogen and so has clean burning characteristics. Its high anti-knock properties, represented by the high octane number, lessen the need to add environmentally harmful anti-knock compounds such as aromatics or lead compounds. Also, unlike gasoline obtained by reforming naphtha or by cracking heavier petroleum fractions, gasoline obtained by alkylation contains few, if any, aromates or olefins, which, environmentally speaking, is a further advantage.

US 5,523,503 discloses the alkylation of saturated hydrocarbons, i.e. isobutane, in a cascade of at least three reactors. During operation, at least two of these reactors are used for the alkylation reaction, in the other at least one reactor the catalyst is regenerated. The alkylation reaction is performed by passing alkylatable compound and alkylation agent to the first reactor, reacting at least a portion of the alkylatable compound with at least a portion of the alkylation agent, thereby forming an alkylate-containing first effluent stream, passing this first effluent stream together with alkylation agent through the second reactor, resulting in an alkylate-containing second effluent stream.

The quality of the alkylate resulting from this prior art process - as characterised by the octane number - still needs improvement, however.

The present invention provides an alkylation process which facilitates an improved quality of alkylate.
In this process an alkylatable compound is reacted with an alkylation agent to form an alkylate, said process being performed in an apparatus comprising a series of at least two reactors, which process comprises the following steps:
a) the alkylatable compound and the alkylation agent are introduced into the first reactor, wherein at least a portion of the alkylatable compound is reacted with at least a portion of the alkylation agent to produce a product stream,
b) step a) is performed at least once more in a downstream reactor employing, instead of said alkylatable compound, a stream comprising said product stream,
   during which process the product stream is subjected at least once to a distillation step to remove at least a portion of the alkylate from the product stream before the product stream is introduced into a downstream reactor.

By way of this interstage distillation, the alkylate concentration is kept at a relatively low level in the entire process. In this way, the amount of undesired by-products that can be formed by, e.g., degradation of alkylate or condensation of degradation products is minimised.

The interstage distillation can be performed by any kind of distillation unit. Preferably, the distillation is performed in a simple way, preferably by using 1-40, more preferably 1-30, even more preferably 1-20, and most preferably 1-10 theoretical plates. This offers a quick and cost-effective way to separate alkylate from the product stream. A specifically suitable distillation technique is flash distillation, which involves only one theoretical plate.
Such a simple distillation will result in an alkylate stream (i.e. the stream containing the alkylate removed from the product stream) also containing at least 5 liquid volume percent (LV%) of alkylatable compound, generally about 5-30 LV%. In other words, the resulting stream of removed compounds preferably contains at least 5 liquid volume percent of alkylatable compound.

In order to obtain an alkylate stream suitably pure for storage or blending, i.e. an alkylate stream containing up to about 0.1 LV% of alkylatable compound, distillation over about 80-100 theoretical plates is required. Such a distillation involves higher costs and is more time consuming than distillation over fewer, e.g. 1-40, theoretical plates. Hence, for interstage distillation, such a profound distillation over 80-100 theoretical plates is less desired.

Preferably, at least 50% of the alkylate initially present in the product stream is removed by interstage distillation. More preferably, at least 60% and most preferably at least 80% is removed. Preferably, after distillation, the product stream contains at least 3% of the initial amount of alkylate.

The conditions, e.g. temperature and pressure, used for interstage distillation will depend on the composition of the product stream. These conditions will depend for instance on the boiling points of the compounds present in the product stream and can easily be determined by the skilled person. For instance, if an alkylate is produced from isobutane and C₄ olefins, removal of C₅⁺-product compounds is desired. If flash distillation is applied for this purpose, the preferred distillation temperature range is 50-100°C. more preferably 70-80°C, at a corresponding pressure range of 0.7-1.5, preferably 1.0-1.2MPa (7-15, preferably 10-12 bar)

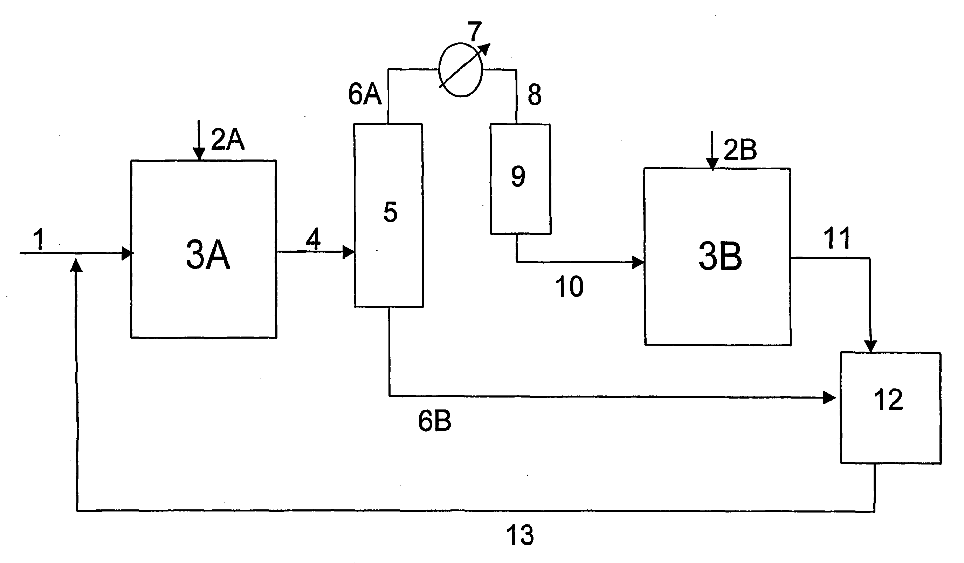

Figure 1 shows a schematic lay-out of an apparatus suitable for carrying out the process according to the invention. By way of example, this figure displays only two reactors with the distillation unit in between. Naturally, it is also possible to use more than two, e.g. three or four, reactors. If only one distillation unit is used, it can be positioned between the first and the second reactor, the second and the third reactor, or the third and the fourth reactor, etc. Alternatively, two distillation units can be used, which can be placed between the first and the second reactor and the second and the third reactor, or between the first and the second reactor and between the third and the fourth reactor, etc. If more than distillation units are used, they can, for instance, be placed between every two reactors. So, in case of four reactors, between the first and the second, between the second and the third, and between the third and the fourth reactor.

The reactors contain a catalyst and are equipped with standard equipment known in the art for measuring temperature, pressure, and flow.
When the reactors contain a solid acid catalyst, this catalyst can for instance be present in a fluidised bed, a fixed bed or suspended in a slurry (e.g. an AKA expanded bed). The reactors may even contain more than one catalyst bed, each with a separate addition of alkylation agent.

The apparatus of Figure 1 comprises reactors 3A and 3B. To reactor 3A, alkylatable compound is fed via duct 1 and the alkylation agent is fed through inlet 2A. The two components react at least partially to produce a product stream. This product stream is subsequently led to distillation unit 5.
The distillation unit may for instance comprise a heater and a pressure reducing device, e.g. a throtting valve, to obtain the preferred temperature and pressure conditions, followed by a distillation device, e.g. a flash drum, where the resulting vapour-liquid mixture is separated into a liquid phase rich in alkylate (the alkylate stream) and a vapour phase rich in alkylatable compound (the product stream).
The alkylate-rich phase leaving the distillation unit is transported via duct 6B to separation unit 12. The rest of the product stream, which is in the vapour phase, is led via duct 6A to condenser 7. Via duct 8 the product stream enters surge drum 9. Losses of alkylatable compound during this separation step can optionally be compensated for by subsequent addition of alkylatable compound to the product stream.
The product stream is transported to reactor 3B, through duct 10. To this reactor alkylation agent is fed through inlet 2B. The alkylation agent can (partially) react with the alkylatable compound present in the product stream. The resulting effluent is transported via duct 11 to separation unit 12.

Separation unit 12 may comprise one or more devices. This separation unit serves to separate alkylatable compound from the effluents fed to the unit. The alkylatable compound can be recycled through duct 13. This stream and, therefore, the alkylatable compound introduced into reactor 3A, may contain minor amounts of other compounds, such as alkylate. Other streams leaving the separation unit comprise the alkylate and usually also comprise a stream containing "lights", such as hydrogen and/or propane, and an n-alkanes-containing stream.

Suitable catalysts for the present process are liquid acid catalysts, such as sulphuric acid and hydrogen fluoride, and solid acid catalysts. At present, in commercial alkylation equipment use is made of liquid acid catalysts. As these liquid acids are toxic and highly corrosive, their use requires high-quality equipment and phase separations in order to remove the liquid acid from the product. Therefore, the use of solid acid catalysts is preferred.

Suitable solid acid catalysts comprise aluminium chloride-containing catalysts such as aluminium chloride promoted with HCl, liquid catalysts on a solid support - e.g. silica-supported trifluoromethanesulfonic or sulfuric acid - zeolite-supported boron trifluoride,
silica-supported antimony pentafluoride, and catalysts comprising a hydrogenating metal component and a solid acid constituent, the latter being preferred.

Examples of suitable hydrogenating metal components are constituents of the transition metals, such as metals of Group VIII of the Periodic Table, or mixtures thereof. Among these, noble metals of Group VIII of the Periodic Table are preferred. Platinum, palladium, and mixtures thereof are especially preferred. The amount of hydrogenating metal component will be dependent on its nature. When the hydrogenating metal component is a noble metal of Group VIII of the Periodic Table, the catalyst generally will contain in the range of 0.01 to 2 wt.% of the metal, preferably 0.1-1 wt.%, calculated as metal.
Examples of solid acid constituents are zeolites such as zeolite beta, MCM-22, MCM-36, mordenite, X-zeolites and Y-zeolites, including H-Y-zeolites and USY-zeolites, non-zeolitic solid acids such as silica-alumina, sulphated oxides such as sulphated oxides of zirconium, titanium, or tin, sulphated mixed oxides of zirconium, molybdenum, tungsten, etc., and chlorinated aluminium oxides. The presently preferred solid acid constituents are zeolites, including mordenite, zeolite beta, X-zeolites and Y-zeolites, including H-Y-zeolites and USY-zeolites, sulphated oxides, and chlorinated aluminium oxides. Mixtures of solid acid constituents can also be employed.
The preferred solid acid catalyst comprises a hydrogenating metal component on a carrier which comprises 2-98 wt.% of solid acid constituent and 98-2 wt.% of a matrix material, calculated on the carrier. Preferably, the carrier comprises 10-90 wt.% of matrix material, and 90-10 wt.% of solid acid constituent. More preferably, the carrier comprises 10-80 wt.% of matrix material and the balance is solid acid constituent.

Especially preferred is the catalyst wherein the carrier comprises 10-40 wt.% of matrix material and the balance is solid acid constituent. In the present specification the term matrix material encompasses all components which are present in the catalyst except for the solid acid constituent and the hydrogenating metal component. Examples of suitable matrix materials are alumina, silica, clays, and mixtures thereof. Matrix materials comprising alumina are generally preferred. A matrix material which consists essentially of alumina is considered most preferred at this point in time.

Preferably, the solid acid catalyst has a particle size of at least 0.5 mm. More preferably, the particle size is at least 0.8 mm, most preferably at least 1.0 mm. The upper limit of the particle size preferably lies at 10 mm, more preferably at 5 mm, even more preferably at 3 mm. In the present specification, the term particle size is defined as the average diameter of the solid part of the catalyst, as will be clear to the skilled person.
The solid acid catalyst can be prepared by processes common to the industry. In the case of catalysts comprising a hydrogenating metal component and a solid acid constituent, the preparation process may comprise, say, shaping the solid acid constituent, after mixing it with a matrix material, to form particles, followed by calcination of the particles. The hydrogenating function can, e.g., be incorporated into the catalyst composition by impregnating the carrier particles with a solution of a hydrogenation metal component.

If the alkylation reaction is performed using a solid acid catalyst, this catalyst may have to be regenerated regularly with hydrogen. The regeneration can be conducted together with the alkylation reaction in the same reactor. It is also possible to separate the alkylation and regeneration steps by switching each reactor between an alkylation and a regeneration mode.
Preferably, two types of regeneration are used: mild regeneration and high-temperature regeneration. Mild regeneration is generally carried out at a temperature in the range of 233 to 523 K and a pressure from 10.0MPa (1 to 100 bar), whereas high-temperature regeneration is usually effected at a temperature of at least 423 K, preferably in the range of 423-873, more preferably 473-673 K.
Mild regeneration of the solid acid catalyst is performed by contacting the catalyst with hydrogen in the presence of hydrocarbon, e.g. the product stream, alkylatable compound, or formed alkylate already present in the reactor. Typically, the hydrogen will be dissolved in the hydrocarbon. Preferably, the solution contains at least 10% of the saturation concentration of hydrogen; said saturation concentration being defined as the maximum quantity of hydrogen which can be dissolved in the hydrocarbon at regeneration temperature and pressure. Depending on the applied feed rates it may be more preferred for the solution to contain at least 50% of the saturation concentration, even more preferably at least 85%. At relatively low feed rates it is generally preferred to have as saturated a solution of hydrogen in the mixture as possible.

The regeneration frequency depends on a number of conditions, including the nature of the catalyst, the reaction and regeneration conditions, and the amount of hydrogen present during the regeneration step. Preferably, mild regeneration is performed before there is any substantial decrease of catalytic activity. Such a decrease can be observed by breakthrough of alkylation agent, which can be measured by analysing the concentration of alkylation agent in the product stream. If mild regeneration is performed prior to breakthrough of alkylation agent, it is possible to obtain a product of nearly constant composition in a high yield. Typically, the mild regeneration frequency is in the range of once per 10 hours to 10 times per hour, preferably once per 3 hours to 3 times per hour, and even more preferably once per 2 hours to 2 times per hour.
To effect a long-term process on a commercial scale one can carry out a high-temperature regeneration after every 50, preferably after every 100 mild regenerations. Pilot plant experiments have shown that it is possible to effect a long-term process when the catalyst is subjected to a high-temperature regeneration after every 200-400 mild regenerations. Depending on the exact process variables on a commercial scale, this value may be higher or lower in actual practice.

In a preferred embodiment the process is performed using a solid acid catalyst and at least four reactors. During operation at least two of these reactors are used for the alkylation, like reactors 3A and 3B in Figure 1. These reactors are said to be in the alkylation mode. In the other at least two reactors the catalyst is mildly regenerated. These reactors are said to be in the regeneration mode. Regularly, the at least two reactors are switched from alkylation mode to mild regeneration mode and *vice versa.* Therefore, the invention is also directed to an alkylation process wherein an alkylatable compound is reacted with an alkylation agent in the presence of a solid acid catalyst to form an alkylate and wherein the catalyst is regenerated, said process being performed in an apparatus comprising a series of at least two reactors in a zone A and a series of at least two catalyst-containing reactors in a zone B, in which process
i. the zone A reactors and the zone B reactors cycle back and forth per zone between alkylation mode and mild regeneration mode,
ii. the alkylation mode comprises introducing an alkylation agent into a first reactor of the zone through which the alkylatable compound passes, reacting a portion of the alkylatable compound with at least a portion of the alkylation agent to produce a product stream, and performing this operation at least once more in a downstream reactor in the same zone employing, instead of said alkylatable compound, a stream comprising said product stream,
iii. the mild regeneration mode comprises contacting the solid acid catalyst in each of the at least two reactors of the zone with hydrogen,
   during which process the product stream is subjected at least once to a distillation step to remove at least a portion of the alkylate from the product stream before the product stream is introduced into a downstream reactor.

More preferably, the interstage distillation is performed between every two reactors in the alkylation mode. This results not only in a reduction of the amount of alkylate present in the downstream reactor(s), but also serves to obtain an almost constant alkylate concentration throughout the entire system. With such an almost constant alkylate concentration, the catalyst in all reactors will deactivate at almost equal rate.

Additionally, the apparatus may comprise one or more reactors which can replace one or more of the at least two reactors in zone A and zone B. Preferably, the apparatus contains one such additional reactor. The availability of such additional reactors makes the process very flexible. If, owing to circumstances, the catalyst in one or more of the reactors deactivates to an unacceptable extent during the process, these one or more reactors can be replaced with one or more additional reactors. The deactivated catalyst can then be regenerated by contacting it at high temperature with hydrogen in the gas phase to recover its original activity without affecting the ongoing alkylation process. After this high-temperature regeneration, the reactor containing the high-temperature regenerated catalyst can serve as said additional reactor.

Preferred alkylatable compounds to be used in the process according to the invention are isoalkanes having 4-10 carbon atoms, such as isobutane, isopentane, isohexane, or mixtures thereof.
Preferred alkylation agents are olefins having 2-10 carbon atoms, preferably 2-6 carbon atoms, more preferably 3-5 carbon atoms, e.g. propene, butene, pentene. The alkylation of isobutane with butene or a mixture of butenes constitutes an attractive embodiment of the process according to the invention.

The process is typically performed under conditions such that at least a portion of the alkylation agent and the alkylatable compound are in the liquid phase or the supercritical phase. In general, the process according to the invention is performed at a temperature in the range of 233 to 523 K, preferably in the range of 293 to 423 K, more preferably in the range of 338 to 368 K, and a pressure in the range of 0.1 to 10.0MPa, preferably 0.5-4.0MPa, more preferably 1.5-3.0MPa (1 to 100 bar, preferably 5 to 40 bar, more preferably 15 to 30 bar). 0.1 to 10.0MPa, preferably 0.5-4.0MPa, more preferably 1.5-3.0MPa

If mild regeneration is performed, it is generally carried out at a temperature in the range of 233 to 523 K and a pressure from0.1-10.0MPa (1 to 100 bar). Although regeneration and alkylation steps can be performed at different temperatures and pressures, it is preferred for the regeneration temperature, expressed in K, and the regeneration pressure not to differ from the reaction temperature and pressure by more than 20%, more preferably not by more than 10%, still more preferably not by more than 5%. Most preferably, the regeneration temperature and pressure and the reaction temperature and pressure are essentially the same.

The molar ratio of alkylatable compound to alkylation agent (I/O) in the total feed in the reactors preferably is higher than 5:1, more preferably higher than 50:1. Higher molar ratios are considered preferred for performance reasons, because they generally yield an increase in octane number and stability. The upper limit for this ratio is determined by the type of process applied, and by the process economics. It is not critical, and may be as high as 5,000:1. Generally, figures of, e.g., 1,000:1 or lower are preferred. These high molar ratios can be obtained in various ways known to the skilled person, e.g., by multiple inlets for alkylation agent or internal recycling of reactor contents. At this moment a molar ratio of alkylatable compound to alkylation agent of 150-750:1 is considered most preferred.

The feed rate (WHSV) of the alkylation agent generally is in the range of 0.01 to 5, preferably in the range of 0.05 to 0.5, more preferably in the range of 0.1 to 0.3 grams of alkylation agent per gram of catalyst per hour.

Preferred alkylates to be produced by the process according to the invention are C₅+ alkylates with a minimum of C₉+ alkylates. The C₅+ alkylate obtained using the process according to the invention preferably has a C₉+ content of less than 30 wt.%, more preferably of less than 20 wt.%, most preferably of less than 10 wt.%.
The process according to the invention makes it possible to obtain a C₅+ alkylate yield in excess of 200%, calculated on the weight of the consumed olefin, preferably of 204% or higher.
The quality of the alkylate product obtained in the process according to the invention can be measured by the RON of the product. The RON is a measure of the anti-knock rating of gasoline and/or gasoline constituents. The higher the RON, the more favourable the anti-knock rating of the gasoline will be. Depending on the type of gasoline engine, generally speaking a higher anti-knock rating is of advantage when it comes to the working of the engine. The product obtained in the process according to the invention preferably has a RON of 90 or higher, more preferably of 92 or higher, most preferably 94 or higher. The RON is obtained by determining, e.g., via gas chromatography, the percentage by volume of the various hydrocarbons in the product. The percentages by volume are then multiplied by the RON contribution and added up.
Examples of compounds with a RON of 90 or higher are isopentane, 2,2-dimethyl butane, 2,3-dimethyl butane, trimethyl butane, 2,3-dimethyl pentane, 2,2,4-trimethyl pentane, 2,2,3-trimethyl pentane, 2,3,4-trimethyl pentane, 2,3,3-trimethyl pentane, and 2,2,5-trimethyl hexane.

### EXAMPLES

Tests were conducted using an apparatus comprising two reactors in the alkylation mode and two in the regeneration mode. Isobutane was used as the alkylatable compound; 1-butene as the alkylation agent. The reaction temperature was 70°C, the WHSV was 0.19 hr⁻¹, and I/O was 24.

Without interstage distillation, the olefin conversion was 81% and the RON of the produced alkylate was 95.3. Using a flash drum, the olefin conversion increased to 100% and the RON of the alkylate was raised to 96.2. This shows the positive effect of interstage distillation.

Further tests have shown that the alkylate concentration in an apparatus with two reactors in the regeneration mode, two reactors in the alkylation mode, and a flash drum between the latter two reactors gives a flat, alkylate concentration profile throughout the reactors.
A comparable result could be reached by using three reactors in the alkylation mode, three reactors in the regeneration mode, and a flash drum between the first and the second and the second and the third reactor in the alkylation mode.

## Claims

1. A process for the alkylation of saturated hydrocarbons wherein an alkylatable compound is reacted with an alkylation agent to form an alkylate, said process being performed in an apparatus comprising a series of at least two reactors, which process comprises the following steps:
a) the alkylatable compound and the alkylation agent are introduced into the first reactor, wherein at least a portion of the alkylatable compound is reacted with at least a portion of the alkylation agent to produce a product stream,
b) step a) is performed at least once more in a downstream reactor employing, instead of said alkylatable compound, a stream comprising said product stream,
during which process the product stream is subjected at least once to a distillation step to remove at least a portion of the alkylate from the product stream before the product stream is introduced into a downstream reactor.

2. A process according to claim 1 wherein the distillation is performed over 1-40 theoretical plates.

3. A process according to claim 2 wherein the distillation is performed over 1-10 theoretical plates.

4. A process according to claim 3 wherein the distillation is flash distillation.

5. A process according to any one of the preceding claims wherein besides alkylate also a portion of the alkylatable compound is removed from the product stream, the resulting stream of removed compounds containing at least 5 liquid volume percent of alkylatable compound.

6. A process according to any one of the preceding claims wherein the at least two reactors contain a solid acid catalyst comprising a solid acid constituent and at least one Group VIII metal.

7. A process according to any one of the preceding claims wherein a series of at least four reactors is used.

8. A process according to any one of the preceding claims wherein the apparatus comprises a series of at least two catalyst-containing reactors in a zone A and a series of at least two catalyst-containing reactors in a zone B, in which process
i. the zone A reactors and the zone B reactors cycle back and forth per zone between alkylation mode and mild regeneration mode,
ii. the alkylation mode comprises steps a) and b) of claim 1, during which the product stream is subjected at least once to an interstage distillation step to remove at least a portion of the alkylate from the product stream before the product stream is introduced into a downstream reactor, and
iii. the mild regeneration mode comprises contacting the solid acid catalyst in each of the at least two reactors of the zone with hydrogen.

9. A process according to any one of the preceding claims wherein the alkylatable compound is isobutane and the alkylation agent comprises C₃-C₅ alkenes.

10. A process according to any one of the preceding claims wherein the process is conducted at a pressure in the range of 0.5-4.0MPa (5-40 ba).

## Patentansprüche

1. Verfahren zur Alkylierung von gesättigten Kohlenwasserstoffen, wobei eine alkylierbare Verbindung unter Bildung eines Alkylats mit einem Alkylierungsmittel umgesetzt wird, wobei das Verfahren in einer Apparatur durchgeführt wird, die wenigstens zwei in Reihe geschaltete Reaktoren umfasst, wobei das Verfahren die folgenden Schritte umfasst:
a) die alkylierbare Verbindung und das Alkylierungsmittel werden in den ersten Reaktor eingeführt, wobei wenigstens ein Teil der alkylierbaren Verbindung mit wenigstens einem Teil des Alkylierungsmittels umgesetzt wird, wobei ein Produktstrom entsteht;
b) Schritt a) wird wenigstens ein weiteres Mal in einem nachgeschalteten Reaktor durchgeführt, wobei anstelle der alkylierbaren Verbindung ein Strom, der den Produktstrom umfasst, eingesetzt wird;
wobei der Produktstrom während des Verfahrens wenigstens einmal einem Destillationsschritt unterzogen wird, so dass wenigstens ein Teil des Alkylats aus dem Produktstrom entfernt wird, bevor der Produktstrom in einen nachgeschalteten Reaktor eingeführt wird.

2. Verfahren gemäß Anspruch 1, wobei die Destillation über 1 bis 40 theoretische Böden durchgeführt wird.

3. Verfahren gemäß Anspruch 2, wobei die Destillation über 1 bis 10 theoretische Böden durchgeführt wird.

4. Verfahren gemäß Anspruch 3, wobei die Destillation eine Entspannungsdestillation ist.

5. Verfahren gemäß einem der vorstehenden Ansprüche, wobei neben Alkylat auch ein Teil der alkylierbaren Verbindung aus dem Produktstrom entfernt wird, wobei der resultierende Strom von entfernten Verbindungen wenigstens 5 Flüssigvolumenprozent alkylierbare Verbindung enthält.

6. Verfahren gemäß einem der vorstehenden Ansprüche, wobei die wenigstens zwei Reaktoren einen festen sauren Katalysator enthalten, der einen festen sauren Bestandteil und wenigstens ein Metall der Gruppe VIII umfasst.

7. Verfahren gemäß einem der vorstehenden Ansprüche, wobei wenigstens vier in Reihe geschaltete Reaktoren verwendet werden.

8. Verfahren gemäß einem der vorstehenden Ansprüche, wobei die Apparatur wenigstens zwei in Reihe geschaltete katalysatorhaltige Reaktoren in einer Zone A und wenigstens zwei in Reihe geschaltete katalysatorhaltige Reaktoren in einer Zone B umfasst, wobei bei diesem Verfahren:
i. die Reaktoren der Zone A und die Reaktoren der Zone B pro Zone zwischen einem Alkylierungsmodus und einem milden Regenerationsmodus hin- und herwechseln;
ii. der Alkylierungsmodus die Schritte a) und b) von Anspruch 1 umfasst, während deren der Produktstrom wenigstens einmal einem Zwischenstufen-Destillationsschritt unterzogen wird, so dass wenigstens ein Teil des Alkylats aus dem Produktstrom entfernt wird, bevor der Produktstrom in einen nachgeschalteten Reaktor eingeführt wird; und
iii. der milde Regenerationsmodus das In-Kontakt-Bringen des festen sauren Katalysators in jedem der wenigstens zwei Reaktoren der Zone mit Wasserstoff umfasst.

9. Verfahren gemäß einem der vorstehenden Ansprüche, wobei es sich bei der alkylierbaren Verbindung um Isobutan handelt und das Alkylierungsmittel C₃-C₅-Alkene umfasst.

10. Verfahren gemäß einem der vorstehenden Ansprüche, wobei das Verfahren bei einem Druck im Bereich von 0,5 bis 4,0 MPa (5 bis 40 bar) durchgeführt wird.

## Revendications

1. Procédé pour l'alkylation d'hydrocarbures saturés où un composé alkylable est mis à réagir avec un agent d'alkylation pour former un alkylat, ledit procédé étant mis en oeuvre dans un appareil comprenant une série d'au moins deux réacteurs, lequel procédé comprend les étapes suivantes :
a) le composé alkylable et l'agent d'alkylation sont introduits dans le premier réacteur, où au moins une partie du composé alkylable est mise à réagir avec au moins une partie de l'agent d'alkylation pour produire un courant de produit,
b) l'étape a) est accomplie au moins une fois encore dans un réacteur en aval employant, à la place dudit composé alkylable, un courant comprenant ledit courant de produit,
procédé pendant lequel le courant de produit est soumis au moins une fois à une étape de distillation pour retirer au moins une partie de l'alkylat du courant de produit avant que le courant de produit soit introduit dans un réacteur en aval.

2. Procédé selon la revendication 1 où la distillation est accomplie sur 1-40 plateaux théoriques.

3. Procédé selon la revendication 2 où la distillation est accomplie sur 1-10 plateaux théoriques.

4. Procédé selon la revendication 3 où la distillation est une distillation flash.

5. Procédé selon l'une quelconque des revendications précédentes où, outre l'alkylat, une partie du composé alkylable est retirée aussi du courant de produit, le courant de composés retirés résultant contenant au moins 5 % en volume liquide de composé alkylable.

6. Procédé selon l'une quelconque des revendications précédentes où les au moins deux réacteurs contiennent un catalyseur acide solide comprenant un constituant acide solide et au moins un métal du groupe VIII.

7. Procédé selon l'une quelconque des revendications précédentes où une série d'au moins quatre réacteurs est utilisée.

8. Procédé selon l'une quelconque des revendications précédentes où l'appareil comprend une série d'au moins deux réacteurs contenant un catalyseur dans une zone A et une série d'au moins deux réacteurs contenant un catalyseur dans une zone B, procédé dans lequel
i. les réacteurs de la zone A et les réacteurs de la zone B parcourent des cycles tour à tour par zone entre un mode d'alkylation et un mode de régénération douce,
ii. le mode d'alkylation comprend les étapes a) et b) selon la revendication 1 pendant lesquelles le courant de produit est soumis au moins une fois à une étape de distillation intermédiaire pour retirer au moins une partie de l'alkylat du courant de produit avant que le courant de produit soit introduit dans un réacteur en aval, et
iii. le mode de régénération douce comprend la mise en contact du catalyseur acide solide dans chacun desdits au moins deux réacteurs de la zone avec de l'hydrogène.

9. Procédé selon l'une quelconque des revendications précédentes où le composé alkylable est l'isobutane et l'agent d'alkylation comprend des C₃-C₅ alcènes.

10. Procédé selon l'une quelconque des revendications précédentes où le procédé est mis en oeuvre à une pression dans la plage de 0,5 - 4,0 MPa (5 - 40 bar).
